# EUROPÄISCHE PATENTSCHRIFT

(11) **EP 0 819 426 B1**
(45) Veröffentlichungstag und Bekanntmachung des Hinweises auf die Patenterteilung: **04.08.2004**
(21) Anmeldenummer: 97109342.2
(22) Anmeldetag: 10.06.1997
(51) Int. Cl.: A61K 7/48, A61K 7/50, B01F 17/00, C08G 77/46, A61K 7/06

(54) **Alpha, Omega-Polyetherpolysiloxane als W/O-Emulgatoren**
Alpha,omega-polyetherpolysiloxanes as W/O emulsifiers
Alpha,oméga-polyétherpolysiloxanes comme émulsifiants E/H

(30) Priorität: 20.06.1996 DE 19624550
(43) Veröffentlichungstag der Anmeldung: 21.01.1998
(73) Patentinhaber: Goldschmidt AG, 45127 Essen (DE)
(72) Erfinder: Floyd, David Thomas, Midlothian, VA 23112 (US); Jenni, Klaus, Dr., 58454 Witten (DE); Leidreiter, Holger, Dr., 45529 Hattingen (DE); Walter, Alfred, 45239 Essen (DE)

(56) Entgegenhaltungen:
- EP-A- 0 266 921
- EP-A- 0 398 177
- EP-A- 0 407 089
- US-A- 4 988 504
- US-A- 5 132 047

## Beschreibung

Die Erfindung betrifft die Verwendung von α,ω-Polyetherpolysiloxanen oxanen als W/O-Emulgatoren, insbesondere in Antiperspirantformulierungen.

Die bekannten und auf dem Markt befindlichen Antiperspirantgele bestehen in der Regel aus
- antiperspirantaktiven Wirkstoffen, wie z. B. Aluminiumchlorid, Aluminiumchlorhydrat, aktive Aluminiumchlorhydrate und Zirkoniumsalze,
- Wasser,
- Ölen, insbesondere Siliconölen, sowie
- Emulgatoren.

Üblicherweise werden diese Formulierungen hergestellt, indem man die Wirkstoffsalze in Wasser löst und diese Lösung als diskontinuierliche Komponente in die Ölphase, die entsprechende W/O-Emulgatoren enthält, unter Rühren einbringt.

Normalerweise sind diese W/O-Formulierungen trübe. Wird aber der Brechungsindex der Wasserphase dem Brechungsindex der Ölphase angeglichen, so entstehen klare W/O-Gele. Üblicherweise werden hierzu der Wasserphase Polyalkohole, wie Zuckeralkohole, oder Polyethylenglykole in Mengen von 25 bis 50 Gew.-% zugegeben. Die benötigte Menge ist abhängig von Art und Menge der Bestandteile der Emulsion und kann leicht durch einfache Versuche festgestellt werden. Weitere Bestandteile der Emulsion, wie Konservierungsmittel, Parfümöle, Farbstoffe und Stabilisatoren, werden in der Regel der Phase zugegeben, in der sie löslich oder leicht dispergierbar sind. Anschließend kann nach Bedarf, z. B. mit Hilfe einer Kolloidmühle, homogenisiert werden.

Die Verwendung von Emulgatorsystemen auf Polysiloxanbasis ist bekannt und beispielsweise in US-A-4 988 504 ausführlich beschrieben. Gegenüber den bis dahin bekannten Polysiloxanemulgatoren werden gemäß US-A-4 988 504 solche vorgeschlagen, die aus
a) Einheiten der Formel

   R₂SiO_{2/2}

   R = Wasserstoff oder ein substituierter oder unsubstituierter Kohlenwasserstoffrest mit 1 bis 12 Kohlenstoffatomen,
b) Einheiten der Formel

   RR¹SiO_{2/2}

   R¹ = Polyalkylenether der Formel

   -Rₐ³-(OR²)ₙ-OR⁴

   - R² =: -CH₂CH₂-,
   - R³ =: substituierte oder unsubstituierte Alkylengruppe mit 1 bis 20 Kohlenstoffatomen,
   - R⁴ =: R,
   - n: hat einen Wert von 5 bis 20 und
   - a: ist 0 oder 1, und
c) Endgliedern der Siloxankette
bestehen, wobei das Molekulargewicht der Komponente a) etwa 25 000 bis 35 000 betragen soll.

Diese Emulgatoren mit seitenständigen Polyoxyalkylengruppen sollen verbesserte Eigenschaften für Antiperspirantsticks in bezug auf Stabilität und niedrigen Wachsanteil bewirken.

Die EP-B-0 407 089 betrifft eine transparente Wasser-in-Silikonöl-Emulsion, geeignet zur äußeren Anwendung auf Säugetierhaut oder -haar, umfassend zusätzlich zu Wasser:
i. 1 bis 50 Gew.-% eines flüchtigen Polydimethylsiloxans;
ii. 0,1 bis 20 Gew.-% eines Silikontensid-Inhaltsstoffs, umfassend ein Polymer von Dimethylpolysiloxan mit Polyoxyethylen- und/oder Polyoxypropylen-Seitenketten mit einem Molekulargewicht von 10 000 bis 50 000 und der Strukturformel: 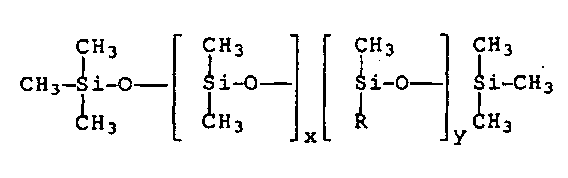 worin R -H oder 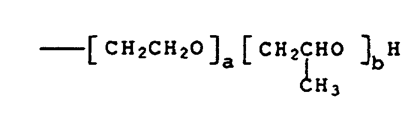 ist,
   a einen Wert von 9 bis 115 hat,
   b einen Wert von 0 bis 50 hat,
   x einen Wert von 133 bis 673 hat,
   y einen Wert von 25 bis 0,25 hat und
iii. 1 bis 50 Gew.-% eines transparenzgebenden Mittels, das mindestens ein mehrwertiger Alkohol ist.

Auch die hier vorgeschlagenen Siloxanemulgatoren weisen demnach Polyoxyethylen- und Polyoxypropylengruppen in der Seitenkette auf und sind durch ein hohes Molekulargewicht gekennzeichnet. Die beschriebenen Emulsionen sollen eine gute Lager- und Temperaturstabilität sowie hautfreundliche Eigenschaften besitzen. Nachteilig bei diesen Siloxanemulgatoren ist jedoch ihre extrem hohe Viskosität, so daß sie nur in Lösemitteln handhabbar sind. Sie werden daher bevorzugt als Dispersion in kurzkettigen (D₄, D₅) Siliconölen eingesetzt, die eine Viskosität unterhalb von 5 mm².s⁻¹ aufweisen müssen. Bevorzugt wird dabei eine 10 %ige Emulgatorformulierung eingesetzt. Der Einsatz dieser Emulgatoren ist damit zwangsläufig mit dem Einbringen kurzkettiger Siliconöle verbunden und bedeutet somit eine Einschränkung in der Auswahl der Komponenten der gewünschten Antiperspirantgele.

Aufgabe der vorliegenden Erfindung ist die Bereitstellung von Emulgatoren für Antiperspirantformulierungen auf der Basis von W/O-Emulsionen, die eine niedrige Viskosität aufweisen und damit ohne den Einsatz von Lösungsmitteln oder Dispersionen handhabbar sind und die gewünschte emulgierende Wirkung in möglichst geringer Konzentration entfalten. Die Emulgatoren sollen hohe Stabilität aufweisen und diese auch bei thermischer Belastung beibehalten. Aufgrund des kosmetischen Anwendungsbereichs müssen die Emulgatoren dabei physiologisch unbedenklich sein.

Erfindungsgemäß wird diese Aufgabe gelöst durch die Verwendung von Copolymerisaten der allgemeinen Formel 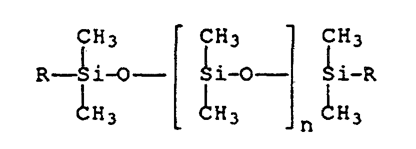
R = -(CH₂-)ₘO-(C₂H₄O-)ₓ(C₃H₆O-)_{y}R¹
   m = 2 bis 4,
   x = 3 bis 100,
   y = 0 bis 50,
   R¹ = H, CH₃, CH₂CH₃,
n = 50 bis 200,
als Emulgatoren in W/O-Emulsionen in Mengen von 0,1 bis 20 Gew.-%, bezogen auf das Gesamtgewicht der Emulsion.

Das Gewichtsverhältnis von Oxyethylen- zu Oxypropylengruppen wird dabei vorzugsweise so gewählt, daß das Gewichtsverhältnis von Polyethylenoxid zu Polypropylenoxid 100 : 10 bis 20 : 80 beträgt.

Besonders bevorzugt sind Copolymerisate, bei denen R¹ = H und n = 100 bis 150 ist und das Gewichtsverhältnis von Polyethylenoxid zu Polypropylenoxid bei 80 : 20 bis 40 : 60 liegt.

Die Oxyethylen- und Oxypropyleneinheiten können statistisch verteilt oder blockweise angeordnet sein.

Der erfindungsgemäße Emulgator ist im Gegensatz zu den bekannten Emulgatoren - an den terminalen Siloxangruppen - mit Polyethergruppen modifiziert. Diese Art von Pfropfcopolymeren weist im Gegensatz zu den üblichen kammartig modifizierten Typen einen geringeren Modifizierungsgrad von zwei Polyethergruppen pro Polymermolekül auf. Durch Modifikation der hydrophoben Reste (Index y und Index n) und der hydrophilen Reste (Index x) im gesamten Molekül sind die erfindungsgemäß verwendeten Polyethersiloxane auf die gewünschten HLB-Werte und notwendigen Grenzflächenaktivitäten einstellbar. Dabei ergeben sich Emulgatoren mit relativ niedrigem Molekulargewicht, mit denen es überraschenderweise gelingt, Emulsionen mit hervorragender Stabilität bezüglich erhöhter Temperatur, Gefrier/Tau-Cyclen und Langzeitlagerung herzustellen. Dabei weist der erfindungsgemäß eingesetzte Emulgator gegenüber dem Stand der Technik eine Reihe von Vorteilen auf. Er ist als 100 %iges Produkt niedrigviskos und gut handhabbar und leichter als definierte Verbindung herstellbar. Im Vergleich zu lösemittelhaltigen Produkten sind geringere Einsatzkonzentrationen erforderlich, was im logistischen Bereich zu erheblichen Einsparungen führt. In den Produkten des Stands der Technik wird Silikonöl verwendet. Demgegenüber ergibt sich als Vorteil für die erfindungsgemäßen Verbindungen, daß bei Bedarf auch silikonölfreie Formulierungen möglich sind.

Die erfindungsgemäßen Emulgatoren eignen sich zur Herstellung von W/O-Emulsionen mit Siliconölen, die linear oder cyclisch sein können. Bevorzugt sind Siliconöle, wie Octamethylcyclotetrasiloxan. Zusammen mit den Siliconölen können weitere Öle oder Wachse emulgiert werden.

Beispiele solcher öligen Substanzen sind synthetische Hautöle, wie Isopropylmyristat, Isopropylstearat, Laurinsäurehexylester, Ölsäureoleylester. Ferner sind flüssige Wachse, wie Jojobaöl, geeignet.

Weitere Bestandteile der öligen Phase können natürliche pflanzliche oder tierische Öle sein, wie z. B. Nerzöl, Fischöl, Klauenöl, Knochenöl, Rizinusöl, Sonnenblumenöl, Avocadoöl, Olivenöl, Erdnußöl, Palmöl. Den zu emulgierenden Ester- und Siliconölen können ferner Mikrowachse oder andere Verdickungsmittel zugesetzt werden, durch welche die Konsistenz der erhaltenen Emulsionen von flüssig bis fest eingestellt werden kann.

Zur Erhöhung der Kältestabilität können der Emulsion Polyalkohole, z. B. Glycerin, 1,2-Propylenglykol, Sorbit, in Mengen von 0,5 bis 15 Gew.-% zugesetzt werden. Die Wärmestabilität kann durch Zusatz von Elektrolyten, z. B. Natriumchlorid, Calciumchlorid und/oder Magnesiumsulfat, verbessert werden. Diese Zusätze werden mit 0,5 bis 10,0 Gew.-% eingesetzt. Darüber hinaus kann durch Zusatz von Zinkstearat oder Aluminiumstearat die Stabilität der Formulierung verbessert werden.
Die Einsatzkonzentration des Emulgators sollte mindestens 1 Gew.-% betragen, die Ölphase kann 15 bis 50 Gew.-% der Gesamtformulierung ausmachen.

Bei einem Wassergehalt von 70 bis 85 Gew.-% hat die Emulsion eine pastöse Konsistenz. Weniger Wasser senkt die Viskosität des Systems bis zu einer lotionähnlichen Konsistenz, und es kann sogar noch weniger Wasser verwendet werden, um eine Emulsion sehr geringer Viskosität herzustellen.

Die Antiperspirant-W/O-Emulsionen werden in üblicher Weise hergestellt, indem man Siliconöle, vorzugsweise die Polysiloxane D₄ und D₅, allein oder in Verbindung mit Hautölen, in dem emulgierenden Mittel, dem Polyethersiloxan, löst und dispergiert. Enthält dieses höherschmelzende Anteile, z. B. Silicon- oder Bienenwachs, wird die ölige Phase über den Schmelzbereich dieser Bestandteile erwärmt.

Die Wasserphase wird dann der öligen Phase unter Rühren langsam zugegeben. Bei transparenten W/O-Emulsionsgelen werden die Brechungsindices der Wasserphase denen der Ölphase angeglichen. Dieses kann durch entsprechende Einstellung des Verhältnisses von Polyalkoholen (Glycerin, Propylenglycol, Sorbit u.ä.) zu Wasser in der Wasserphase geschehen.

### Beispiele

### 1. Emulgatoren

Die Tabelle zeigt den chemischen Aufbau einiger Emulgatoren, die für die erfindungsgemäße Anwendung geeignet sind:

**Tabelle 1:**

| erfindungsgemäße Emulgatoren | | | | |
|---|---|---|---|---|
| | R' | n | (C₂H₄O-)ₓ(C₃H₆O-)_{y}R' mittleres angestrebtes Molekulargewicht [g/mol] | Gewichtsverhältnis C₂H₄O : C₃H₆O |
| Emulgator 1 | H | 50 | 1 600 | 58 : 42 |
| Emulgator 2 | H | 150 | 400 | 100 : 0 |
| Emulgator 3 | H | 100 | 1 400 | 42 : 58 |
| Emulgator 4 | H | 200 | 500 | 60 : 40 |
| Emulgator 5 | CH₃ | 100 | 2 550 | 42 : 58 |
| Emulgator 6 | H | 100 | 1 400 | 12 : 88 |

### 2. Formulierungsbeispiele

### 2.1. Herstellung

Die Tabelle 2 zeigt die Zusammensetzung erfindungsgemäßer Emulsionen bzw. Emulsionsgele.

Die Herstellung der Emulsion erfolgt in einem Behälter, der mit einem Rührer versehen ist. In den Behälter werden die Bestandteile A gegeben und ggf. aufgeschmolzen. Unter Rühren wird das Wasser, in welchem ggf. Kochsalz, Propylenglykol, je nach Rezeptur Aluminiumsalze (in Lösung) gelöst sind, zu dem Gemisch aus Emulgator und Öl gegeben.
Bei klaren, gelartigen Formulierungen ist der Brechungsindex der wäßrigen Phase durch Zusatz von Polyalkoholen (z. B. Propylenglycol, Glycerin oder Sorbitol) dem Brechungsindex der Ölphase angeglichen.

Die erhaltenen W/O-Emulsionen bzw. -Emulsionsgele können nach Bedarf z. B. mit einer Kolloidmühle oder einem Homogenisator, der nach dem Druckprinzip arbeitet, homogenisiert werden. Die hergestellten Emulsionen zeigen Teilchengrößen < 1 µ Teilchendurchmesser.

### 2.2. Emulsionsbeispiele 1 bis 7

Die Emulsionen 1, 3, 4, 5, 6, 7 haben cremeartige bzw. gelartige Konsistenz, die Emulsion 2 ist flüssig. Die Emulsionen bzw. die Emulsionsgele sind 3 Monate, bei 20 °C und 45 °C gelagert, stabil. Die Emulsionen zeigen nach 5 x Abkühlen auf -15 °C und darauffolgendem Erwärmen auf 20 °C keine Beeinträchtigung der Stabilität. Auch nach 6monatiger Lagerung der Emulsionen bei 20 °C ist kein Viskositätsabbau festzustellen.

Bei den Beispielformulierungen 2 und 5 handelt es sich um Hautpflegeemulsionen. Die Emulsionen haben ein glattes und glänzendes Erscheinungsbild. Sie lassen sich sehr gut auf der Haut verteilen, ziehen schnell ein und vermitteln ein angenehmes, samtartiges, leicht kühlendes Hautgefühl.

Bei den Beispielformulierungen 1, 3, 4, 6 und 7 handelt es sich um transparente Emulsionen für den Einsatz als Antiperspirant. Die Transparenz dieser Formulierungen wird durch Anpassung des Brechungsindex der wäßrigen Phase an den der öligen Phase erreicht. Diese Formulierungen lassen sich gut auf der Haut verteilen und hinterlassen ein samtartiges Gleitgefühl auf der Hautoberfläche.

**Tabelle 2:**

| Emulsionsformulierungen | | | | | | | |
|---|---|---|---|---|---|---|---|
| Emulsionsbeispiel Nr. | 1 | 2 | 3 | 4 | 5 | 6 | 7 |
| Bestandteile | Gew.-% | Gew.-% | Gew.-% | Gew.-% | Gew.-% | Gew.-% | Gew.-% |
| Emulgator 1 | 3, 0 | - | - | - | - | - | - |
| Emulgator 2 | - | 4,0 | - | - | - | - | - |
| Emulgator 3 | - | - | 1,5 | - | - | - | - |
| Emulgator 4 | - | - | - | 3,0 | 4,0 | - | - |
| Emulgator 5 | - | - | - | - | - | 1,0 | - |
| Emulgator 6 | - | - | - | - | - | - | 1,5 |

| Phase A) | | | | | | | |
|---|---|---|---|---|---|---|---|
| Octylmethylcyclotetrasiloxan | 13,0 | - | 7,2 | 6,5 | - | 7,5 | 7,0 |
| Decamethylcyclopentasiloxan | - | - | 7,3 | 6,5 | - | 7,5 | 7,5 |
| Isopropylmyristate | - | - | - | - | 8,0 | - | - |
| Paraffinöl DAB 8 | - | 12,5 | - | - | 13,0 | - | - |
| Avocadoöl | - | - | - | - | 1,0 | - | - |
| Cetyl Dimethicone *1) | - | - | - | - | 0,5 | - | - |
| Caprylic/Capric-Triglyceride | - | 12,5 | - | - | - | - | - |
| Zinkricinoleate + Tetrahydroxypropylethylendiamin + Laureth-3 + 1,2-Propylenglykol *2) | - | - | - | 2,5 | - | - | - |

| Phase B) | | | | | | | |
|---|---|---|---|---|---|---|---|
| Wasser | 19,0 | 70,3 | 5,0 | 16,5 | 67,0 | 20,0 | 20,0 |
| NaCl | - | 0,7 | - | - | 2,0 | - | - |
| 1,2-Propylenglykol | 25,0 | - | 22,0 | 25,0 | 4,5 | 24,0 | 24,0 |
| Aluminiumchlorhydratlösung *3) | 40,0 | - | - | 40,0 | - | 40,0 | 40,0 |
| Aluminium-Zirkonium-Tetrachlorohydrat-Glycin-Komplex *4) | - | - | 57,0 | - | - | - | |
| Summe | 100,0 | 100,0 | 100,0 | 100,0 | 100,0 | 100,0 | 100,0 |

| | | | | | | | |
|---|---|---|---|---|---|---|---|
| *1) ABIL Wax 9801 (Polysiloxan-Polyalkylen-Copolymer), Th. Goldschmidt AG | | | | | | | |
| *2) TEGO Deo CW 90 (Deodorant-Wirkstoff), Th. Goldschmidt AG | | | | | | | |
| *3) Reach 501 (Antiperspirant-Wirkstoff, 50 % in Wasser), Reheis | | | | | | | |
| *4) Reach 36GLY (Antiperspirant-Wirkstoff, 36 % in Wasser), Reheis | | | | | | | |

### 3. Formulierungsbeispiele

### 3.1. Emulgatoren

Zum Vergleich wird das am Markt angebotene Produkt "Formulation aid DC 3225 C", Dow Corning, eingesetzt. Hierbei handelt es sich um eine trübe Dispersion eines kammartig modifizierten Polydimethylsiloxans mit Polyethylenglykol/Polypropylenglykolgruppen als Modifikation (Pfropfcopoplymer) in Octamethylcyclotetrasiloxan und Decamethylcyclopentasiloxan. Aus dieser Dispersion setzt sich bereits nach kurzer Zeit ein heller Bodensatz ab.

Bei den erfindungsgemäßen Emulgatoren handelt es sich hingegen um klare und homogene Produkte. Separationserscheinungen wie bei dem Vergleichsprodukt des Marktes sind bei den erfindungsgemäßen Emulgatoren nicht beobachtet worden.

### 3.2. Formulierungen

Die Rezepturen in Tabelle 3 werden hergestellt wie vorher beschrieben.

**Tabelle 3:**

| Formulierungsbeispiele | | | | | |
|---|---|---|---|---|---|
| Formulierungsbeispiel | Antiperspirant-Gele | | Hautpflegeemulsionen | | |
| | 3 | 8 | 9 | 10 | 11 |
| Bestandteile | Gew.-% | Gew.-% | Gew.-% | Gew.-% | Gew.-% |
| Emulgator 3 | 1,5 | - | - | - | - |
| Emulgator 4 | - | - | 3,0 | - | 3,0 |
| DC 3225 C | - | 15,0 | - | 30,0 | - |

| Phase A) | | | | | |
|---|---|---|---|---|---|
| Octamethylcyclotetrasiloxan | 7,2 | 1,0 | - | - | 13,5 |
| Decamethylcyclopentasiloxan | 7,3 | - | - | - | 13,5 |
| Isopropylmyristate | - | - | 8,0 | 8,0 | 8,0 |
| Paraffinöl DAB 8 | - | - | 13,0 | - | - |
| Avocadoöl | - | - | 1,0 | 1,0 | 1,0 |

| Phase B) | | | | | |
|---|---|---|---|---|---|
| Wasser | 5,0 | 5,0 | 71,0 | 59,0 | 59,0 |
| NaCl | - | - | 2,0 | 2,0 | 2,0 |
| 1,2-Propylenglykol | 22,0 | 22,0 | 2,0 | - | - |
| Aluminium-Zirkonium-Tetrahydro-Glycin-Komplex *4) | 57,0 | 57,0 | - | - | - |
| Summe | 100,0 | 100,0 | 100,0 | 100,0 | 100,0 |

| | | | | | |
|---|---|---|---|---|---|
| *4) Reach 36GLY (Antiperspirant-Wirkstoff, 36 % in Wasser), Reheis | | | | | |

### 3.3. Vergleich: Viskosität von Gelformulierungen

| | | |
|---|---|---|
| Formulierungsbeispiel | 3 | 8 |
| Viskosität | 45 000 mPas | 49 000 mPas |

Die Viskositäten der Rezepturbeispiele 3 und 8 sind ähnlich.

### 3.4. Vergleich: Silikonölanteil in Hautpflegeformulierungen

| | | | |
|---|---|---|---|
| Formulierungsbeispiel | 9 | 10 | 11 |
| Siliconölanteil in % | 0 % | 27 % | 27 % |

Der Siliconölanteil in den Rezepturbeispielen wird durch den Emulgator festgelegt. Das Vergleichsprodukt des Marktes, DC 3225 C, enthält 89 % Siliconöle. Wie das Beispiel 10 zeigt, ist es nicht möglich, mit DC 3225 C Emulsionen herzustellen, die kein Siliconöl enthalten. Mit den erfindungsgemäßen Emulgatoren ist es hingegen möglich, sowohl siliconölfreie Formulierungen (Beispiel 9) als auch solche Formulierungen herzustellen, die die gleiche Menge an Siliconölen enthalten wie die Formulierungen, die mit DC 3225 C hergestellt werden können (Beispiel 11).

### 4. Untersuchungen zur Wirksamkeit von Antiperspirantformulierungen

### 4.1. Testrezepturen

Die in Tabelle 4 aufgeführten Formulierungen werden zur Bestimmung der Wirksamkeit in allen Untersuchungen eingesetzt. Der Gehalt an Aluminium ist in allen diesen Formulierungen 6 %.

**Tabelle 4:**

| Testrezepturen für Wirksamkeitsbestimmungen | | |
|---|---|---|
| Formulierungsbeispiele | 12 | 13 |
| Emulgator 3 | 1,1 % | - |
| DC 3225 C | - | 10,0 % |
| Octamethylcyclotetrasiloxan | 15,9 % | 7,0 % |
| Aluminiumchlorhydratlösung *3) | 50,0 % | 50,0 % |
| Propylenglykol | 16,0 % | 16,0 % |
| Wasser, destilliert | 17,0 % | 17,0 % |
| Summe | 100,0 % | 100,0 % |

| | | |
|---|---|---|
| *3) Reach 501 (Antiperspirant-Wirkstoff, 50 % in Wasser), Reheis | | |

### 4.2. Freigabe von Aluminium

In den untersuchten Formulierungen liegt der aktive Inhaltsstoff (Aluminiumchlorhydratlösung) als Lösung in der internen, wäßrigen Phase vor. Um wirksam zu werden, muß die Aluminiumchlorhydratlösung die externe, ölige Phase durchtreten. Die Geschwindigkeit der Freigabe des aktiven Inhaltsstoffes (Aluminiumchlorhydratlösung) wird in den Formulierungsbeispielen 12 und 13 untersucht.

Die Formulierungen werden dazu mit 95 % Wasser verdünnt. Nach Schütteln für 5 Minuten wird der Aluminiumgehalt in einer aliquoten Menge der wäßrigen Lösung bestimmt. Dieser 5-Minuten-Wert markiert den Startpunkt. Die Verdünnungen werden weiter geschüttelt, und nach jeweils 1 Stunde und 24 Stunden werden erneut aliquote Mengen zur Analyse abgenommen. Der Gehalt an Aluminium in der wäßrigen Lösung wird durch Atomabsorptionsspektroskopie bestimmt.

Die Ergebnisse sind in Tabelle 5 zusammengefaßt. Es wird deutlich, daß die Formulierungen 12 und 13 eine gleich hohe Freigabegeschwindigkeit von Aluminium aufweisen.

**Tabelle 5:**

| Freigabe von Aluminium | | |
|---|---|---|
| Beispielformulierung | 12 | 13 |
| | % Aluminium wiedergefunden | |
| 5 Minuten | 15 | 16 |
| 1 Stunde | > 90 | > 90 |
| 24 Stunden | > 90 | > 90 |

### 4.3. In-vivo-Test auf Antiperspiranteffektivität

### 4.3.1.Voruntersuchungen (Auswahl der Probanden)

Probanden mit ähnlicher Intensität der Perspiration (Schweißentwicklung) auf der volaren Oberfläche des Unterarms werden ausgewählt.

Dazu werden zunächst die entsprechenden Hautflächen mit Alkohol abgetupft. Dies geschieht zur Entfernung von Restfeuchtigkeit. Eine Mischung aus 3 % Jod in 95 %igem Alkohol wird dann auf die gesamte Fläche aufgetragen. Nach Trocknung der Oberfläche wird eine Suspension aus Stärke und Castor-Öl (50 : 50 w/w) auf die Haut aufgetragen. Die Probanden werden in einem klimatisierten Raum einer Temperatur von 37 °C und einer relativen Feuchtigkeit von 35 % ausgesetzt. Die Dauer des Tests richtet sich dabei nach der Entwicklung von Schweiß bei dem einzelnen Probanden. Eine alternative Prozedur kann darin bestehen, daß die Probanden durch schnelles Gehen zur Entwicklung von Perspiration angeregt werden.

Durch die Perspiration wird Wasser freigesetzt. Bei Anwesenheit von Wasser bildet sich aus Jod und Stärke eine intensiv blau gefärbte Einschlußverbindung (Jod-Stärke-Komplex). Die behandelten Flächen werden durch Inaugenscheinnahme auf ihre Gleichheit untersucht.

### 4.3.2.Behandlung mit der Antiperspirantformulierung

Zur Behandlung der Hautoberfläche mit den Testformulierungen 12 und 13 wird die Technik der Okklusion durch Eintauchen angewandt (Occlusion by Immersion Technique, OBIT).

Die zu behandelnden Hautflächen werden zunächst mit Wasser abgespült. Kleine Plastikbecher (2,5 cm Durchmesser) mit einem Loch (1 cm Durchmesser) im Boden werden mit einem Klebeband auf der Hautfläche befestigt. Dies geschieht bei 12 Probanden mit jeweils 2 bis 3 Bechern auf jedem Unterarm. Durch die Öffnung der Becher werden aliquote Mengen der Probelösung von 1,0 bis 1,4 ml aufgebracht. Nach einer Kontaktzeit von 30 Minuten wird diese Behandlung abgeschlossen. Dann werden die Becher entfernt und überschüssiges Antiperspirantmittel mit Wasser abgespült. Zur späteren Identifizierung werden die behandelten Flächen markiert.

### 4.3.3. Bestimmung der Antiperspirantwirksamkeit

30 Minuten und 4 Stunden nach der Behandlung wird eine Bestimmung der Wirksamkeit der Antiperspirantformulierung durchgeführt. Dazu werden zunächst die entsprechenden Hautflächen mit Alkohol abgetupft. Dies geschieht zur Entfernung von Restfeuchtigkeit. Eine Mischung aus 3 % Jod in 95 %igem Alkohol wird dann auf die gesamte Fläche aufgetragen. Nach Trocknung der Oberfläche wird eine Suspension aus Stärke und Castor-Öl (50 : 50 w/w) auf die Haut aufgetragen. Die Probanden werden in einem klimatisierten Raum bei einer Temperatur von 37 °C und einer relativen Feuchtigkeit von 35 % zum Schwitzen angeregt. Die Dauer dieser Behandlung richtet sich dabei nach der Entwicklung von Schweiß bei jedem einzelnen Probanden. Die behandelten Flächen werden dann visuell untersucht und die Antiperspirantaktivität mit einer Skala 0 bis 100 % bewertet. Die Zahlen stehen für den Anteil an Schweißdrüsen, die durch die Behandlung mit dem Antiperspirantmittel keinen Schweiß absondern.

Basierend auf dem obengenannten Testdesign wird jeweils im Mittel durch jede der Formulierungen 12 und 13 nach 30 Minuten und 4 Stunden eine Reduktion der Perspiration um 20 bis 30 % erreicht.

Das Beispiel zeigt, daß mit den erfindungsgemäßen Emulgatoren hochwirksame Antiperspirantformulierungen hergestellt werden können.

Die mit den erfindungsgemäß zu verwendenden Emulgatoren hergestellten W/O-Emulsionen eignen sich auch für technische Zwecke, z. B. zur Verwendung als Pflegemittel und Polituren für Möbel, lackierte Bleche und Fußböden.

## Patentansprüche

1. Verwendung von Copolymerisaten der allgemeinen Formel 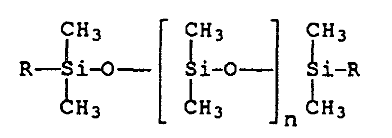
R = -(CH₂-)ₘO-(C₂H₄O-)ₓ(C₃H₆O-)_{y}R¹
m = 2 bis 4,
x = 3 bis 100,
y = 0 bis 50,
R¹ = H, CH₃, CH₂CH₃,
n = 50 bis 200,
als Emulgatoren in W/O-Emulsionen in Mengen von 0,1 bis 20 Gew.-%, bezogen auf das Gesamtgewicht der Emulsion.

## Claims

1. Use of copolymers of the general formula 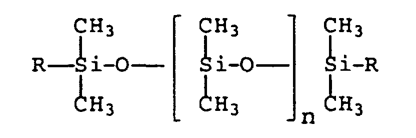
R = -(CH₂-)ₘO-(C₂H₄O-)ₓ(C₃H₆O-)_{y}R¹
m = 2 to 4,
x = 3 to 100,
y = 0 to 50,
R¹ = H, CH₃, CH₂CH₃,
n = 50 to 200,
as emulsifiers in W/O emulsions in amounts from 0.1 to 20% by weight, based on the total weight of the emulsion.

## Revendications

1. Utilisation de copolymères de formule générale 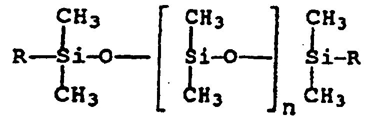
R = -(CH₂-)ₘO-(C₂H₄O-)ₓ(C₃H₆O-)_{y}R¹
m = 2 à 4
x = 3 à 100
y = 0 à 50
R¹ = H, CH₃, CH₂CH₃,
n = 50 à 200
comme émulsifiants dans des émulsions E/H en des quantités de 0,1 à 20% en poids par rapport au poids total de l'émulsion.
